(19) Europäisches Patentamt
European Patent Office
Office européen des brevets

(11) Publication number: **0 068 385**
**B1**

(12) **EUROPEAN PATENT SPECIFICATION**

(45) Date of publication of patent specification: **24.09.86**

(51) Int. Cl.⁴: **C 08 G 18/50,** C 08 G 18/77, C 08 G 77/42, C 08 G 77/46, A 61 L 17/00

(21) Application number: **82105425.1**

(22) Date of filing: **21.06.82**

# (54) Thermoplastic elastomers for medical use as moulded articles brought into direct contact with blood.

(30) Priority: **22.06.81 JP 97270/81**
**27.04.82 JP 72298/82**

(43) Date of publication of application:
**05.01.83 Bulletin 83/01**

(45) Publication of the grant of the patent:
**24.09.86 Bulletin 86/39**

(84) Designated Contracting States:
**CH DE FR GB LI**

(56) References cited:
**DE-A-2 909 430**
**DE-B-1 260 150**
**FR-A-2 168 221**
**US-A-4 057 595**

(73) Proprietor: **KANEGAFUCHI KAGAKU KOGYO KABUSHIKI KAISHA**
**2-4 Nakanoshima 3-chome**
**Kita-ku Osaka-shi Osaka-fu (JP)**

(72) Inventor: **Kira, Kazuaki**
**12-33-1303, 1-chome, Ryugadai Suma-ku**
**Kobe-shi Hyogo-ken (JP)**

(74) Representative: **Vossius & Partner**
**Siebertstrasse 4 P.O. Box 86 07 67**
**D-8000 München 86 (DE)**

**The file contains technical information submitted after the application was filed and not included in this specification**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 068 385 B1

**Description**

Antithrombogenic elastomers are required for the manufacture of molded articles which are brought into direct contact with blood, particularly artificial hearts, and other artificial organs. More specifically, they are used for making, for example, blood-vessel catheters, monitoring tubes, blood bags, extracorporeal circulation circuits such as artificial kidneys and mechanical hearts and lungs, A—V shunts, blood bypass tubes, artificial hearts, auxiliary artificial hearts, blood pumps, and balloon pumps. These applications essentially require the use of elastomers which have excellent antithrombogenic properties, mechanical properties (for example mechanical strength, elasticity and durability) and moldability.

Specific examples of the antithrombogenic elastomers known in the art include general-purpose high molecular materials, such as soft vinyl chloride polymers, polyurethanes and silicone rubbers, segmented polyurethanes such as Biomer® of Ethicon, U.S.A., heparinized polyurethane elastomers, which are disclosed in Japanese Patent Publication No. 13729/80, and copolymers of a polysiloxane and a polyurethane bonded directly to each other by nitrogen and silicon, which are disclosed in U.S. Patent No. 3,562,352.

The general-purpose high molecular materials are, however, unsatisfactory in antithrombogenic properties. The segmented polyurethane is inferior in antithrombogenic properties, though it has high mechanical strength. The heparinized polyurethane elastomer is very low in antithrombogenic properties after it has released heparin, and as heparin is physiologically active, this elastomer involves a lot of complications in molding and sterilization, and is, therefore, costly. The copolymer disclosed in U.S. Patent No. 3,562,352, which is known under the trade name of Avcothane®, is superior to any other known material in antithrombogenic properties, and is often used clinically. It is, however, inferior in its mechanical properties to, for example, the segmented polyurethane. Moreover, its antithrombogenic properties depend largely on the molding conditions, and are not always satisfactory. It is manufactured by a process which comprises mixing solutions of a polyurethane and a polysiloxane having reactive terminal groups and reacting them as shown by the following reaction equation:

$$R_1—O—CO—NH—R_2+CH_3COO—Si(X)_2—O—R_3 \rightarrow R_1—O—CO—\underset{\underset{\textstyle Si(X)_2—O—R_3}{|}}{N}—R_2+CH_3COOH$$

in which $R_1$ and $R_2$ each stand for a polyurethane chain segment, $R_3$ stands for a polysiloxane chain, and X stands for a substituent bonded to a silicon atom. As is obvious from this equation, it is in substance a graft copolymer. Since a polysiloxane obviously has at least two reactive terminal groups, it is a thermosetting resin containing a polysiloxane group as a crosslinking agent. This resin is, therefore, moldable by a limited number of methods, such as coating and dipping. The limitation to the molding method necessitates storage of the resin in the form of a solution. If the polyfunctional terminal groups of the polyurethane and the polysiloxane react with each other during storage, the solution becomes highly viscous or gelatinous and therefore unusable.

Thus, there has hitherto not been any antithrombogenic elastomer that is excellent in antithrombogenic and mechanical properties, and moldable by a wide range of methods.

It is an object of this invention to provide thermoplastic elastomers for use in medical applications as molded articles having excellent and reliable antithrombogenic and mechanical properties.

As a result of extensive research it has been found that molded articles of specific polyurethanes or polyurethane ureas containing an organic silicone polymer in the main chain and containing a polyether or a polyester segment having a molecular weight of 500 to 6,000 as a soft segment other than said organic silicone polymer are superior in antithrombogenic and mechanical properties, and may be prepared by a wide variety of molding methods.

This invention relates to thermoplastic elastomers for medical use as molded articles brought into direct contact with blood comprising a polyurethane or a polyurethane urea containing 4 to 15 weight percent of an organic silicone polymer having a molecular weight of 500 to 10,000 in the main chain, and containing a polyether or a polyester segment having a molecular weight of 500 to 6,000 as a soft segment, other than said organic silicone polymer.

The organic silicone polymer is a polymer having a molecular weight of 500 to 10,000, preferably 700 to 3,000, as determined by the vapor pressure equilibrium method or by osmometry. Although there is no particular limitation to the method of incorporating the organic silicon polymer, it is preferred to employ a polysiloxane in order to ensure the antithrombogenic properties of the inventive elastomer. Methylphenylpolysiloxane, fluoroalkylmethylpolysiloxane and polydimethylsiloxane can, for example, be employed. Polydimethylsiloxane is particularly preferred. The most preferred polydimethylsiloxane which may be incorporated has the following formula:

$$—R_1(OR_2)_a(OR_3O)_b(\underset{CH_3}{\overset{CH_3}{|}}\!\!\!\!\!\!\overset{}{Si}—O)_c\underset{CH_3}{\overset{CH_3}{Si}}(OR_4O)_d(R_5O)_eR_6—$$

in which $R_1$ to $R_6$ each stand for an alkylene group having at least one carbon atom, a and e are each an integer of 0 to 30, preferably 0 to 20, b and d are each 0 or 1, and c is an integer of from 6 to 134. $R_1$ to $R_6$ are preferably ethylene, propylene, butylene or hexamethylene groups.

If a long period of use *in vivo* is contemplated, it is preferred that b and d represent 0, so that there may not be formed any Si—O—C bond that is easy to hydrolyze. The letter c represents an integer which depends on the molecular weight of polydimethylsiloxane which may be 500 to 10,000, and preferably 700 to 3,000.

The elastomer of this invention has a soft segment ratio of preferably 40 to 80% by weight, and most preferably 50 to 70% by weight, in order to maintain satisfactory mechanical-properties of the molded articles. The soft segment ratio is calculated in accordance with the following formula:

$$\frac{\text{Total molecular weight of soft segments}}{\text{Total molecular weight of elastomer}} \times 100\ (\%)$$

An elastomer having a soft segment ratio which is lower than 40% by weight is likely to be deficient in elasticity, while an elastomer having a soft segment ratio exceeding 80% by weight is likely to be inferior in mechanical strength such as tensile strength.

The soft segments are segments between urethane or urea bonds, or between urethane and urea bonds, having a molecular weight of at least 500 as determined by the vapor pressure equilibrium method or by osmometry, and a glass transition temperature not exceeding ordinary room temperature. Examples of the soft segments include polysiloxanes, polyethers, polyesters, and their block copolymers. The preferred soft segments, apart from polysiloxanes, are polyethers or polyesters having a molecular weight of 500 to 6,000. More preferred are polyether segments having a high degree of hydrolysis stability *in vivo* and having a molecular weight of 700 to 3,000, particularly having the formula

$$-\!\!\left(CH_2-CH_2-CH_2-CH_2-O\right)\!\!-_{26\ \text{to}\ 30} \text{ and}$$

$$-\!\!\left(CH_2-\underset{}{\overset{CH_3}{\overset{|}{C}H}}-O\right)\!\!-_{16\ \text{to}\ 20}.$$

The elastomer of this invention is preferably an essentially thermoplastic elastomer composed of linear macromolecules which do not contain any crosslinked structure.

The elastomer of this invention has an intrinsic viscosity [η] of preferably 0.2 to 2.0 dl/g, and more preferably 0.5 to 1.5 dl/g, measured in dioxane at 32°C.

The elastomer of this invention will yield under appropriate molding conditions molded articles with a substantially smooth surface. The term "substantially smooth surface" as herein used means a surface of which the examination by a scanning electron microscope of 1,000 magnifications does not reveal any physical unevenness, provided, however, that any and all unevenness created by a mold surface, foreign matter, or other factors not directly associated with the nature of the elastomer *per se* is disregarded. The appropriate molding conditions mean dependence on any ordinarily conceivable means for smooth surface formation, for example, the use of a homogeneous solution of the elastomer in a good solvent for molding by coating. If such appropriate molding conditions are employed, the elastomer of this invention does not form any physically uneven surface, though it may form a microstructure of chemical phase separation as an ordinary block copolymer does. If a medical instrument is formed from a molded product obtained under such appropriate molding conditions which enable formation of a substantially smooth surface, it will be highly antithrombogenic.

The elastomers of this invention may be manufactured by a process which will hereinafter be described. It is most preferred to manufacture the elastomers by reacting an isocyanate compound, a compound having groups containing active hydrogen atoms and containing soft segments having a molecular weight of 500 to 6,000, and a synthetic component having active hydrogen atoms and/or isocyanate groups and containing in its main chain an organic silicone polymer, and if required, by employing a chain extender. It is possible to apply an ordinary process for manufacturing a thermoplastic polyurethane elastomer. The organic silicon content and soft segment ratio of the elastomer to be prepared are first selected, and the quantity of the synthetic component is chosen to satisfy them. Then the synthetic component is added into a solvent, dissolved and reacted. Although all of the synthetic components may be added together, it is preferred to react an isocyanate compound with a compound having active hydrogen atoms and containing soft segments having a molecular weight of 500 to 6,000 first to form a prepolymer having terminal isocyanate groups, and react it with the synthetic component having groups containing active hydrogen atoms and containing an organic silicon polymer in the main chain, followed by incorporation of a chain extender, if required. Alternatively, the prepolymer is first reacted with a chain extender and then with the synthetic component having groups containing active hydrogen atoms and containing an organic silicon polymer in the main chain.

It is preferred to add continuously and slowly the synthetic component having groups containing

active hydrogen atoms and containing an organic silicone polymer in the main chain, and a highly reactive diamine as a chain extender. The reaction is performed while heating or in the presence of a catalyst. Although any catalyst of the type used for the urethane synthesis may be used, it is preferred to use triethylenediamine or other amines, diazabicycloundecane, or other catalysts which can be removed before molding, since the elastomer is used for medical purposes.

Although it is possible to use any isocyanate compound of the type used for the manufacture of polyurethanes, diisocyanates are particularly preferred. The preferred examples of the diisocyanates include tetramethylene diisocyanate, hexamethylene diisocyanate, cyclohexane - 1,4 - diisocyanate, 2,4-tolylene diisocyanate, 2,6-tolylene diisocyanate, a mixture of 2,4-tolylene diisocyanate and 2,6-tolylene diisocyanate, xylylene diisocyanate, 4,4'-diphenylmethane diisocyanate, 1,4-phenylene diisocyanate, 1,3-phenylene diisocyanate, and naphthalene - 1,5 - diisocyanate, and mixtures thereof. It is also possible to use any isocyanate compound obtained from regenerated compounds.

As regards the compound having groups containing active hydrogen atoms and containing soft segments having a molecular weight of 500 to 6,000, it is possible to use any compound of the type used for reaction with isocyanates to form polyurethanes. It is preferred to use diol compounds, such as polyethers, polyesters and polycaprolactones. It is more preferred to use a diol compound of the polyether series having a high degree of hydrolysis stability *in vivo*, and a molecular weight of 700 to 3,000, for example, polyethylene ether glycol, polypropylene ether glycol, polytetramethylene ether glycol, a polyethylene oxide—polypropylene oxide—polyethylene oxide copolymer, or a mixture thereof. Compounds of the formulas

$$HO\!-\!(CH_2-CH_2-CH_2-CH_2-O)_{26\text{ to }30}H$$

and

$$HO\!-\!(CH_2-\underset{}{\overset{CH_3}{\overset{|}{CH}}}-O)_{16\text{ to }20}H$$

are particularly preferred.

Preferred examples of the diol compounds of the polyester series include polyesters obtained by polycondensation of ethylene glycol, propylene glycol, butylene glycol, diethylene glycol, pentamethylene glycol, cyclohexane-1,4-diol, cyclohexane-1,4-dimethanol, or other glycols, or a mixture thereof, with adipic acid, maleic acid, succinic acid, phthalic acid, isophthalic acid, terephthalic acid, or other dibasic acids, or their derivatives such as acid esters or halides, and mixtures of such polyesters.

As regards the synthetic component containing an organic silicone polymer in its main chain, it is preferable to use a compound containing an organic silicone polymer in its main chain, and having two functional groups selected from active hydrogen and isocyanate groups. The preferred examples of the organic silicone polymer include polydimethylsiloxane, methylphenylpolysiloxane and fluoroalkylmethylpolysiloxane. The preferred functional group is a group containing active hydrogen atoms. The preferred examples of groups having active hydrogen atoms include carbinol, amino and mercapto groups, or a mixture thereof. A particularly preferred synthetic component containing an organic silicon polymer in the main chain is represented by the following formula:

$$XR_1\!-\!(OR_2)_a\!-\!(OR_3O)_b\!-\!(\underset{\underset{CH_3}{|}}{\overset{\overset{CH_3}{|}}{Si}}-O)_c\underset{\underset{CH_3}{|}}{\overset{\overset{CH_3}{|}}{Si}}\!-\!(OR_4O)_d\!-\!(R_5O)_eR_6X$$

in which $R_1$ to $R_6$ each stand for an alkylene group having at least one carbon atom, X stands for a group containing active hydrogen, a and e each stand for an integer of 0 to 30, b and d are each 0 or 1, and c is an integer of from 6 to 134. More preferably, $R_1$ to $R_6$ are each an ethylene, propylene, butylene or hexamethylene group, X is a hydroxyl or amino group, a and e are both an integer of 0 to 20, and b and d are both zero, since the elastomer should preferably not contain any easily hydrolyzable Si—O—C bond if used for a long period *in vivo*. The integer for which c stands depends on the molecular weight of polydimethylsiloxane, which is 500 to 10,000, and preferably 700 to 3,000.

If any chain extender is employed, it is suitable to use a chain extender having two active hydrogen groups, for example, aliphatic diamines such as ethylenediamine, propylenediamine, butylenediamine and hexamethylenediamine, alicyclic and aliphatic-aromatic diamines such as cyclohexanediamine, piperazine and xylylene diamine, aromatic diamines such as tolylenediamine, phenylenediamine and 4,4'-diaminodiphenylmethane hydrazines, glycols such as ethylene glycol and 1,4-butanediol, or water.

Suitable examples of the solvent to be used include N,N-dimethylformamide, N,N-dimethylacetamide, dimethylsulfoxide, N-methylpyrrolidone, dioxane and tetrahydrofuran. In order to completely dissolve the synthetic component and the resulting polymer, it is preferred to use a mixture of a low polar solvent such as dioxane or tetrahydrofuran, and a high polar solvent such as N,N-dimethylacetamide or N,N-dimethylformamide.

The elastomer synthesized as hereinabove described may be used either in the form of a solution or in the form of pellets which are obtained by precipitating the elastomer in water or the like, washing the precipitate carefully with water, ethanol or the like to remove impurities, and drying it.

The elastomer of this invention was tested for antithrombogenic properties *in vitro* in accordance with the Lee White method (Izumi Kanai and Masamitsu Kanai: Outline of Clinical Inspections, VI-81, Kinbara Publishing Co., 1970). It showed an improvement over a segmented polyurethane elastomer in total blood coagulation time, and was found superior in antithrombogenic properties. Samples molded under varying conditions were tested, and all found highly reliable in their antithrombogenic properties.

The elastomers of this invention were also found excellent in their mechanical properties.

An antithrombogenic elastomer is generally required to have a tensile strength of at least 100 $kg/cm^2$, and an elongation of at least 300 to 500%. The elastomers of this invention showed a tensile strength of 100 to 500 $kg/cm^2$, and an elongation of at least 500%.

The elastomers of this invention in the form of a solution lend themselves to molding by coating, dipping or casting. Pellets are moldable by any ordinary molding method, for example, extrusion, injection or press molding. Whether in the form of dry pellets or a solution, the elastomers of this invention show a high degree of storage stability, are easy to handle, and show a high degree of reproducibility.

The test results confirm the usefulness of the antithrombogenic elastomers according to this invention for forming the exposed surfaces of medical instruments or devices which are brought into direct contact with blood. More specifically, they are useful for making, for example, artificial hearts, pumping chambers for auxiliary circulation devices, balloon pumps, extracorporeal circuits for auxiliary circulation devices such as artificial kidneys and mechanical hearts and lungs, blood bags, and catheters.

The invention will now be decribed in further detail with reference to several examples.

Example 1

A vessel which has been fully dried and purged with nitrogen, is charged with 54.7 parts by weight of polytetramethylene ether glycol having a molecular weight of 2,000. It is dehydrated at a temperature of 90°C and a reduced pressure not higher than 0.1 mm Hg for 30 minutes. After the temperature of the glycol has been adjusted to 50°C, 250 parts by weight of a solvent mixture of dehydrated and purified dioxane and N,N-dimethylacetamide in a weight ratio of 7:3 are added. Then 27.35 parts by weight of 4,4'-diphenylmethane diisocyanate are added and dissolved under stirring. Diazabicycloundecene in a quantity of 0.05% by weight of 4,4'-diphenylmethane diisocyanate is added as a catalyst, and stirring is continued for 30 minutes. Then, 4.75 parts by weight of ethylene glycol are added, and the reaction is carried out for 30 minutes. The reaction is continued by dropping slowly a solution of 13.2 parts by weight of a terminal carbinolpolydimethylsiloxane in 150 parts by weight of a solvent mixture of dehydrated and purified dioxane and N,N-dimethylacetamide in a weight ratio of 7:3. The polydimethylsiloxane has the formula

$$HO\left(CH_2CH_2O\right)_a\left(\underset{CH_3}{\overset{CH_3}{\underset{|}{\overset{|}{Si}}}}-O\right)_b\left(CH_2CH_2O\right)_cH$$

in which the sum of a and c has an average value of 30.2, and b has an average value of 14.2. The siloxane has a molecular weight of about 2,400. The polymer solution thus obtained is poured in a large quantity of water, whereby the polymer is precipitated. The polymer is carefully washed with water, then dried and washed with ethanol in a Soxhlet extractor, whereby an antithrombogenic elastomer according to this invention is obtained.

The elastomer shows an intrinsic viscosity of 0.86 dl/g in dioxane at 32°C. The elastomer is dissolved in a solvent mixture of dioxane and N,N-dimethylacetamide in weight ratio of 7:3. A film having a thickness of about 0.1 mm is cast from the elastomer solution. The mechanical properties of the film are determined by an apparatus called Shimazu Autograph IS 2000. The film is found to have a smooth surface when it is examined by a scanning electron microscope of 1,000 to 5,000 magnification.

The antithrombogenic properties of the elastomer are examined by the following method. The elastomer is dissolved in a solvent mixture of dioxane and N,N-dimethylacetamide in a weight ratio of 7:3 to form an elastomer solution having an elastomer content of about 5% by weight. This elastomer solution is coated on the inner surface of a test tube having an inside diameter of 10 mm and a length of 100 mm. About 1 ml of fresh blood which had just been collected is placed in the test tube, and the time for coagulation of the blood is examined at 37°C.

For comparison purposes, similar tests are conducted on a segmented polyurethane (Comparative Example 1), and a test tube coated with a solution thereof, as well as a test tube on which no coating had been applied. The solution is prepared by dissolving the segmented polyurethane in a solvent mixture of dioxane and N,N-dimethylacetamide in a weight ratio of 7:3. The solution has a polymer content of 5% by weight.

The test results are shown in Table 1. They confirm the superiority of the elastomer according to this invention in antithrombogenic and mechanical properties.

TABLE 1

| | Antithrombogenic test—blood coagulation time (min.) | Mechanical properties | |
|---|---|---|---|
| | | Tensile strength ($kg/cm^2$) | Elongation (%) |
| Example 1 | 68 to 72 | 350 | 670 |
| Comparative Example 1 | 30 to 38 | 400 | 550 |
| Test tube (glass) not coated | 10 to 13 | — | — |

A transparent, strong and elastic sheet having a thickness of 1 mm is prepared by press molding the elastomer of this invention at a temperature of 200°C and a pressure of 200 $kg/cm^2$ for 15 minutes.

The antithrombogenic properties of this sheet are examined by the following method.

A 3×3 cm elastomer sheet is placed on a watch glass having a cover and maintained at 37°C. A certain quantity of dog's ACD blood (i.e., noncoagulating preservable blood prepared by adding sodium citrate, glucose, etc.) is placed on the sheet and the watch glass, and an aqueous solution of calcium chloride is added to the blood. The weight of coagulated blood (thrombus) is measured after certain time intervals. A ratio of thrombus formation is calculated by the following equation:

$$\text{Ratio of thrombus formation (\%)} = \frac{\text{Weight of thrombus formed on sample after a certain period of time}}{\text{Final weight of thrombus formed on the glass}} \times 100$$

For comparison purposes, similar tests are conducted on a similarly formed sheet of segmented polyurethane elastomer of Comparative Example 1, and on ordinary glass. The results are shown in Table II. They confirm the superiority in antithrombogenic properties of the sheet from the elastomer according to this invention.

TABLE II
Ratio of thrombus formation

| | Duration of contact with blood (min.) | | | | |
|---|---|---|---|---|---|
| | 3 | 5 | 7 | 10 | 15 |
| Sheet of elastomer according to this invention | 17.0% | 26.9% | 32.7% | 37.7% | 43.2% |
| Segmented polyurethane sheet | 25.4% | 44.7% | 55.2% | 65.5% | 77.2% |
| Glass | 61.5% | 85.2% | 96.9% | 99.5% | 99.5% |

The sheet of the elastomer according to this invention was examined by a scanning electron microscope of 1,000 to 5,000 magnification, and found to have a smooth surface.

Comparative Example 1

A segmented polyurethane elastomer is prepared by repeating the procedures of Example 1, except that no terminal carbinolpolydimethylsiloxane is employed, and that the quantity of 4,4'-diphenylmethane diisocyanate is correspondingly reduced by 1.4 parts by weight.

Example 2

A four-necked flask, which has been dried and purged with nitrogen, is charged with 52 parts by weight of polytetramethylene ether glycol having a molecular weight of 2,000. It is dehydrated at a temperature of 95°C and a reduced pressure not higher than 0.1 mm Hg for 30 minutes. After the temperature of the glycol has been adjusted to 45°C, 200 parts by weight of a solvent mixture of dehydrated and purified tetrahydrofuran and N,N-dimethylacetamide in a weight ratio of 8:2 are added. Furthermore 14 parts by

weight of 4,4'-diphenylmethane diisocyanate are added and dissolved. Diazabicycloundecene is added as a catalyst in a quantity of 0.03% by weight relative to 4,4'-diphenylmethane diisocyanate, and stirring is performed for 30 minutes. Then, 18 parts by weight of 4,4'-diphenylmethane diisocyanate and 6 parts by weight of ethylene glycol are added, and the reaction is performed for one hour. After the reaction temperature has been adjusted to room temperature (about 15°C), the reaction is continued by adding continuously a solution of 10 parts by weight of double terminal 3-aminopropylpolydimethylsiloxane having a molecular weight of about 2,000 in 200 parts by weight of a solvent mixture of dehydrated and purified tetrahydrofuran and N,N-dimethylacetamide in a weight ratio of 8:2. There is obtained a transparent, viscous polymer solution. A large quantity of water is added to the solution to precipitate the polymer. After the polymer has been carefully washed with water and dried, it is washed with ethanol in a Soxhlet extractor to yield an antithrombogenic elastomer according to this invention. This elastomer has an intrinsic viscosity [η] of 0.75 dl/g in dioxane at 32°C. The antithrombogenic properties, tensile strength and elongation of the elastomer are examined in accordance with the procedures of Example 1, except that a solvent mixture of tetrahydrofuran and N,N-dimethylacetamide in a weight ratio of 8:2 is employed.

For comparison purposes, similar tests are conducted on segmented polyurethane of Comparative Example 2 and a test tube coated with a solution therof, as well as a test tube on which no such coating had been applied.

The test results are shown in Table III. They confirm the superiority of the elastomer according to this invention in both antithrombogenic and mechanical properties.

Comparative Example 2

A segmented polyurethane elastomer is prepared by repeating the procedure of Example 2, except that no double terminal 3 - aminopropylpolydimethylsiloxane is employed, and that the quantity of 4,4'-diphenylmethane diisocyanate (later added) is correspondingly reduced by 1.25 parts by weight.

TABLE III

| | Antithrombogenic test—blood coagulation time (min.) | Mechanical properties | |
|---|---|---|---|
| | | Tensile strength ($kg/cm^2$) | Elongation (%) |
| Example 2 | 50 to 60 | 430 | 630 |
| Comparative Example 2 | 30 to 38 | 450 | 550 |
| Test tube (glass) not coated | 9 to 12 | — | — |

Example 3

Fifty-eight parts by weight of dehydrated polypropylene ether glycol having a molecular weight of 1,000, 6.4 parts by weight of double terminal 3-hydroxypropylpolydimethylsiloxane having a molecular weight of about 1,000, and 32 parts by weight of 4,4'-diphenylmethane diisocyanate are dissolved in 300. parts by weight of a solvent mixture of dehydrated and purified tetrahydrofuran and N,N-dimethylacetamide in a weight ratio of 8:2. Diazabicycloundecene is added to the solution in a quantity of 0.01% by weight relative to 4,4'-diphenylmethane diisocyanate, and the reaction is performed at 60°C for one hour, whereby a prepolymer having terminal isocyanate groups is obtained. After the reaction temperature has been lowered to room temperature (about 15°C), 300 parts by weight of dehydrated and purified N,N-dimethylacetamide are added to the prepolymer solution to dilute it. The reaction is continued by adding slowly 300 parts by weight of a solution of 3.6 parts by weight of ethylenediamine in dehydrated and purified N,N-dimethylacetamide. The procedure of Example 1 is then repeated for the treatment of the polymer solution to produce a purified elastomer. This elastomer has an intrinsic viscosity of 0.56 dl/g in dioxane at 32°C. The antithrombogenic properties, tensile strength and elongation of the elastomer are examined in accordance with the procedures of Example 1, except that N,N-dimethylacetamide is employed. The elastomer coated on a glass test tube shows a coagulating time of 50 to 65 minutes.

The tensile strength is 470 $kg/cm^2$ and the elongation is 600%.

**Claims**

1. Thermoplastic elastomers for medical use as molded articles brought into direct contact with blood comprising a polyurethane or a polyurethane urea containing 4 to 15 weight percent of an organic silicone polymer having a molecular weight of 500 to 10,000 in the main chain, and containing a polyether or a polyester segment having a molecular weight of 500 to 6,000 as a soft segment other than said organic silicone polymer.

2. The elastomers of claim 1, wherein the organic silicone polymer has a molecular weight of 700 to 3,000.

3. The elastomers of claim 1, wherein the elastomers have a soft segment content of from 40 to 80 weight percent.

4. The elastomers of claim 3, wherein the elastomers have a soft segment content of from 50 to 70 weight percent.

5. The elastomers of claim 1, wherein the polyether segment has the formula:

$$\left(CH_2-\overset{\displaystyle CH_3}{\overset{|}{C}H}-O\right)_{16\sim20}$$

6. The elastomers of claim 1, wherein the polyether segment has the formula:

$$\left(CH_2-CH_2-CH_2-CH_2-O\right)_{26\sim30}$$

7. The elastomers of claim 1, wherein the organic silicone polymer is a polysiloxane.

8. The elastomers of claim 7, wherein the polysiloxane is a polydimethylsiloxane.

9. The elastomers of claim 8, wherein the polydimethylsiloxane has the formula:

$$-R_1\left(OR_2\right)_a\left(OR_3O\right)_b\left(\underset{\displaystyle CH_3}{\overset{\displaystyle CH_3}{Si}}-O\right)_c\underset{\displaystyle CH_3}{\overset{\displaystyle CH_3}{Si}}\left(OR_4O\right)_d\left(R_5O\right)_eR_6-$$

in which $R_1$ to $R_6$ each stand for an alkylene group having at least one carbon atom, *a* and *e* are each an integer of from 0 to 30, *b* and *d* are each 0 or 1, and *c* is an integer of from 6 to 134.

10. The use of thermoplastic elastomers as claimed in any one of claims 1 to 9 for preparing molded articles in medical applications brought into direct contact with blood.

**Patentansprüche**

1. Thermoplastische Elastomere zur medizinischen Verwendung als unmittelbar mit Blut in Kontakt gebrachte Formteile, enthaltend ein Polyurethan oder einen Polyurethan-Harnstoff, die in der Hauptkette 4 bis 15 Gewichtsprozent eines organischen Silikon-Polymeren mit einem Molekulargewicht von 500 bis 10 000 und als weiches Segment ein Polyäther- oder Polyester-Segment mit einem Molekulargewicht von 500 bis 6000, welches nicht dem organischen Silikon-Polymer entspricht, enthalten.

2. Elastomere nach Anspruch 1, in denen das organische Silikon-Polymer ein Molekulargewicht von 700 bis 3000 aufweist.

3. Elastomere nach Anspruch 1, in denen der Gehalt der Elastomere an weichem Segment 40 bis 80 Gewichtsprozent beträgt.

4. Elastomere nach Anspruch 3, in denen der Gehalt der Elastomere an weichem Segment 50 bis 70 Gewichtsprozent beträgt.

5. Elastomere nach Anspruch 1, in denen das Polyäther-Segment die folgende Formel aufweist:

$$\left(CH_2-\overset{\displaystyle CH_3}{\overset{|}{C}H}-O\right)_{16\sim20}$$

6. Elastomere nach Anspruch 1, in denen das Polyäther-Segment die folgende Formel aufweist:

$$\left(CH_2-CH_2-CH_2-CH_2-O\right)_{26\sim30}$$

7. Elastomere nach Anspruch 1, in denen das organische Silikon-Polymer ein Polysiloxan ist.

8. Elastomere nach Anspruch 7, in denen das Polysiloxan ein Polydimethylsiloxan ist.

9. Elastomere nach Anspruch 1, in denen das Polydimethylsiloxan die folgende allgemeine Formel hat:

$$-R_1\left(OR_2\right)_a\left(OR_3O\right)_b\left(\underset{\displaystyle CH_3}{\overset{\displaystyle CH_3}{Si}}-O\right)_c\underset{\displaystyle CH_3}{\overset{\displaystyle CH_3}{Si}}\left(OR_4O\right)_d\left(R_5O\right)_eR_6-$$

in der $R_1$ bis $R_6$ jeweils einen Alkylenrest mit mindestens einem Kohlenstoffatom bedeuten, a und e jeweils

eine ganze Zahl mit dem Wert 0 bis 30 darstellen, b und d jeweils den Wert 0 oder 1 aufweisen und c eine ganze Zahl von 6 bis 134 ist.

10. Verwendung von thermoplastischen Elastomeren nach jedem der Ansprüche 1 bis 9 zur Herstellung von Formteilen, die in medizinischen Anwendungen in unmittelbaren Kontakt mit Blut gebracht werden.

**Revendications**

1. Elastomères thermoplastiques destinés à des utilisations médicales sous la forme d'articles moulés mis en contact direct avec le sang, comportant un polyuréthane ou un composé urée-polyuréthane renfermant 4 à 15% en poids d'un polymère de silicone organique d'un poids moléculaire de 500 à 10.000 dans la chaîne principale, et renfermant un segment de polyéther ou de polyester d'un poids moléculaire de 500 à 6.000 comme segment souple, autre que ledit polymère de silicone organique.

2. Elastomères selon la revendication 1, dans lesquels le polymère de silicone organique a un poids moléculaire de 700 à 3.000.

3. Elastomères selon la revendication 1, dans lesquels ces élastomères ont une teneur en segments souples de 40 à 80% en poids.

4. Elastomères selon la revendication 3, dans lesquels ces élastomères ont une teneur en segments souples de 50 à 70% en poids.

5. Elastomères selon la revendication 1, dans lesquels le segment de polyéther répond à la formule:

$$\left( CH_2-\underset{}{\overset{CH_3}{\overset{|}{C}H}}-O \right)_{16\sim20}$$

6. Elastomères selon la revendication 1, dans lesquels le segment de polyéther répond à la formule:

$$\left( CH_2-CH_2-CH_2-O \right)_{26\sim30}$$

7. Elastomères selon la revendication 1, dans lesquels le polymère de silicone organique est un polysiloxane.

8. Elastomères selon la revendication 7, dans lesquels le polysiloxane est un polydiméthylsiloxane.

9. Elastomère selon la revendication 8, dans lesquels le polydiméthylsiloxane répond à la formule:

$$-R_1\left(OR_2\right)_a\left(OR_3O\right)_b\left(\underset{CH_3}{\overset{CH_3}{Si}}-O\right)_c\underset{CH_3}{\overset{CH_3}{Si}}\left(OR_4O\right)_d\left(R_5O\right)_eR_6-$$

dans laquelle $R_1$ à $R_6$ représentent chacun un groupe alkylène comportant au moins un atome de carbone, *a* et *e* sont chacun un entier de 0 à 30, *b* et *d* valent chacun 0 ou 1, et *c* est un entier de 6 à 134.

10. Application des élastomères thermoplastiques selon l'une quelconque des revendications 1 à 9 à la préparation d'articles moulés pour applications médicales mis en contact direct avec le sang.